# EUROPÄISCHE PATENTSCHRIFT

(11) **EP 2 663 345 B1**
(45) Veröffentlichungstag und Bekanntmachung des Hinweises auf die Patenterteilung: **12.10.2016**
(21) Anmeldenummer: 12700307.7
(22) Anmeldetag: 10.01.2012
(51) Int. Cl.: A61M 1/16

(54) **DIALYSEVERSORUNGSSYSTEM**
DIALYSIS SUPPLY SYSTEM
SYSTÈME D'ALIMENTATION POUR DIALYSE

(30) Priorität: 10.01.2011 DE 102011008223; 10.01.2011 US 201161431224 P
(43) Veröffentlichungstag der Anmeldung: 20.11.2013
(73) Patentinhaber: Fresenius Medical Care Deutschland GmbH, 61352 Bad Homburg (DE)
(72) Erfinder: WEHMEYER, Wolfgang, 72076 Tübingen (DE); KÖHLER, Dietmar, 97633 Aubstadt (DE); BRANDL, Matthias, 97631 Bad Königshofen (DE); GLASER, Benedict, 97422 Schweinfurt (DE)
(74) Vertreter: Herrmann, Uwe
(86) Internationale Anmeldenummer: PCT/EP2012/000085
(87) Internationale Veröffentlichungsnummer: WO 2012/095301

(56) Entgegenhaltungen:
- EP-A1- 0 613 688
- WO-A1-2006/074429
- WO-A2-2004/009158
- DE-A1- 4 203 905
- DE-A1- 19 933 223
- DE-A1-102006 050 272
- DE-A1-102009 058 681
- US-A1- 2009 101 550
- US-A1- 2010 192 686

## Beschreibung

Die vorliegende Erfindung betrifft ein Dialyseversorgungssystem gemäß dem Oberbegriff des Anspruchs 1 mit wenigstens einer zentralen Herstellungseinheit, mittels derer Dialyseflüssigkeit herstellbar ist, mit wenigstens einem Leitungsmittel, mittels dessen Dialyseflüssigkeit förderbar ist, mit mehreren Dialysebehandlungseinheiten und mit Anschlussmitteln, mittels derer eine Dialysebehandlungseinheiten an das Leitungsmittel anschließbar sind.

In einigen Einrichtungen von Dialysezentren sind zentrale Versorgungssysteme zur Bereitstellung von Dialyseflüssigkeit oder Dialyseteilkonzentraten installiert. Die zentral hergestellten Flüssigkeiten oder Flüssigkonzentrate werden über ein Leitungsversorgungssystem zu den einzelnen Behandlungsstationen, z. B. Behandlungsplätzen mit einem Behandlungsmonitor zur Durchführung der Dialysebehandlung, geliefert. An den Behandlungsstationen wird Dialyseflüssigkeit verwendet, um das in einem extrakorporalen Blutkreislauf zirkulierende Blut zu reinigen. Alternativ wird das über die Versorgungsleitung gelieferte flüssige Konzentrat verwendet, um an der Behandlungsstation mit weiteren Lösungskomponenten und Verdünnungsmittel eine behandlungsfertige Dialyseflüssigkeit herzustellen und zur Blutbehandlung freizugeben. Die nach der Blutbehandlung verbrauchte Dialyseflüssigkeit wird über ein Rücklaufrohrsystem des Versorgungskreislaufs abgeleitet und kann z. B. zentral verworfen werden. Die verbrauchte Dialyseflüssigkeit kann aber auch direkt in ein Ablaufsystem gegeben werden.

Durch die zentralen Systeme, die mit Dialyseflüssigkeit bzw. Dialysekonzentrat arbeiten, sind in den Dialysemaschinen der Behandlungsstation (sog. "bedside monitors") gegenüber gängigen Dialysemaschinen wesentliche Teile zur Dialysatherstellung nicht notwendig. Die zentrale Versorgungsstation kann bereits erwärmtes Dialysat zur Behandlungsstation liefern, so dass keine Temperiereinheiten notwendig sind oder diese mit geringeren Leistungen ausgelegt sein können. Ebenso kann auf Entgasungseinheiten in der Dialysemaschine verzichtet werden, da diese Funktion bereits von der Zentralversorgungseinheit übernommen werden kann.

Die Einrichtung von "bedside monitor" Systemen in Dialysebehandlungszentren stellt die Anforderung nach regelmäßiger Desinfizierung des Versorgungssystems und der einzelnen Behandlungsstationen. Im Falle einer defekten "bedside station" bzw. Behandlungsstation können beispielsweise Kontaminationen in das Rohrleitungssystem gelangen und verteilt werden. Zur Sicherheit müssen genaue Desinfektionsprogramme eingehalten werden, die gewährleisten, dass das gesamte Versorgungssystem nicht kontaminiert wird. Abhängig von der Größe und Art der Konstruktion des Rohrleitungssystems kann diese Desinfektion zeitintensiv und mit durchaus großem Aufwand verbunden sein.

Die DE 42 03 905 A2 beschreibt eine Einrichtung zur zentralen Versorgung von Dialysestationen mit Dialysekonzentrat. Um der Gefahr der Kontamination des zentralen Dialysetanks zu begegnen, wird hier ein Sterilfilter zwischen Dialysestation und dem Ringleitungsversorgungssystem geschaltet.

Die US 2010/0181235 A1 befasst sich ebenfalls mit der Problematik einer Rückkontamination bei zentralen Dialyseversorgungssystemen. Hierzu wird ein Puffertank vorgesehen, der ein Zurückfließen kontaminierter Flüssigkeit in das Versorgungsleitungssystem verhindert. Zusätzlich können Ultrafilter in dem Fluidkreislauf auf der Dialysatseite der Behandlungsstation vorhanden sein.

Ferner ist aus der EP 0 436 855 A1 ein Desinfektionsverfahren für eine Dialysebehandlungsstation und einem Ringleitungssystem zur Versorgung der Dialysebehandlungsstation mit Frischwasser bekannt. Dabei wird die Dialysemaschine über eine Ventilschaltung von dem Frischwassersystem abgekoppelt und durch Hitze oder chemische Zusätze eine Desinfektion der Dialysemaschine, die hierzu im Zirkulationsmodus betrieben wird, durchgeführt.

Es ist daher die Aufgabe der vorliegenden Erfindung, ein Dialyseversorgungssystem der eingangs genannten Art in vorteilhafter Weise weiterzubilden, insbesondere dahingehend, dass dieses einfacher, sicherer und hygienischer zu betreiben ist, wobei weiter insbesondere die Desinfektion vereinfacht und/oder eine Kontamination des Rohrleitungssystems durch eine "bedside" Behandlungsstation vermieden werden kann.

Diese Aufgabe wird erfindungsgemäß gelöst durch ein Dialyseversorgungssystem mit den Merkmalen des Anspruchs 1. Danach ist vorgesehen, dass ein Dialyseversorgungssystem mit wenigstens einer zentralen Herstellungseinheit, mittels derer Dialyseflüssigkeit mittelbar und/oder unmittelbar bereitstellbar und/oder herstellbar ist, mit wenigstens einem Leitungsmittel, mittels dessen Dialyseflüssigkeit leitbar ist, mit wenigstens einer Dialysebehandlungseinheit und mit wenigstens einem Anschlussmittel versehen ist, mittels dessen die Dialysebehandlungseinheit mittelbar und/oder unmittelbar an das Leitungsmittel anschließbar ist, sowie mit wenigstens einem Ventil versehen ist, vorzugsweise einem Ventilsystem, mittels dessen der Zufluss und/oder Abfluss von Dialyseflüssigkeit zur Dialysebehandlungseinheit, Spül-, Reinigungs- und/oder Desinfektionsflüsse in verschiedenen Betriebszuständen der Dialysebehandlung und/oder der Anlagendesinfektion steuerbar und/oder regelbar ist, insbesondere im Zusammenhang mit einer Steuerungs- und/oder Regelungseinheit oder durch eine Steuerungs- und/oder Regelungseinheit steuerbar und/oder regelbar ist, wobei als Mittel zur Führung, Förderung, Verarbeitung, Mischung und/oder Bilanzierung der Dialyseflüssigkeit in der Dialysebehandlungseinheit wenigstens ein Hydraulik-Modul vorgesehen ist und wobei das Hydraulik-Modul als Einwegartikel bzw. Disposable ausgeführt ist.

Erfindungsgemäß ist vorgesehen, dass als einzige Mittel zur Führung der Dialyseflüssigkeit in den Dialysebehandlungseinheiten Hydraulik-Module vorgesehen sind, die als Einwegartikel ausgeführt sind.

Das Hydraulik-Modul kann ein UF-Modul sein.

Das Dialyseversorgungssystem kann ein zentrales Dialyseversorgungssystem sein, etwa ein Dialyseversorgungssystem in einem Krankenhaus bzw. der Dialysestation eines Krankenhauses oder einem Dialysezentrum mit einer zentralen Herstellungseinheit zur Bereitstellung von Dialysatflüssigkeit.

Grundsätzlich ist denkbar, dass Teile des Dialyseversorgungssystem stationär angeordnet werden. So kann beispielsweise vorgesehen sein, die zentrale Herstellungseinheit zur Bereitstellung von Dialysatflüssigkeit zentral und ortsfest in dem Gebäude z. B. des Dialysezentrums oder der Dialysestation anzuordnen, während die Dialysebehandlungseinheiten grundsätzlich als mobile Einheiten, vorzugsweise als mobile und verfahrbare Behandlungsmonitore ausgeführt sein können.

Der Begriff Dialyseflüssigkeit oder Dialysat ist hier nicht einengend zu verstehen und umfasst dabei insbesondere nicht nur gebrauchsfertige Dialyselösungen, sondern auch Dialysekonzentrate oder Dialyselösungen, die noch mit weiteren Zusätzen, etwa direkt am Behandlungsplatz versehen werden sollen. So kann beispielsweise eine dialysatführende Leitung eine Leitung sein, die eine gebrauchsfertige Dialyselösungen führt, aber auch eine Leitung, die Bestandteile einer Dialyselösung führt, wie z. B. Dialysekonzentrat.

Das Leitungsmittel kann beispielsweise eine zentrale Ringleitung sein, mittels derer die Dialyseflüssigkeit bereitstellbar und förderbar ist. Über das Anschlussmittel können die Dialysebehandlungseinheiten mittelbar und/oder unmittelbar an das Leitungsmittel angeschlossen werden. Das Anschlussmittel kann hierzu mit entsprechenden Dichtungsmitteln und/oder Kopplungsmitteln versehen sein oder hiermit in Verbindung stehen. Auch kann vorgesehen sein, dass das Anschlussmittel mit einem Ventil, wie z. B. dem Ventil bzw. einem Ventil des Ventilsystems versehen ist, mittels dessen der Zulauf von Dialyseflüssigkeit zur Dialysebehandlungseinheit sperrbar und/oder freigebbar ist.

Das Ventil, mittels dessen der Zufluss und/oder Abfluss von Dialyseflüssigkeit zur Dialysebehandlungseinheit, Spül-, Reinigungs- und/oder Desinfektionsflüsse in verschiedenen Betriebszuständen der Dialysebehandlung und/oder der Anlagendesinfektion steuerbar und/oder regelbar ist, kann im Zusammenhang mit einer Steuerungs- und/oder Regelungseinheit oder durch eine Steuerungs- und/oder Regelungseinheit steuerbar und/oder regelbar sein. Dabei kann es sich um eine dezentrale Steuerungs- und/oder Regelungseinheit, etwa eine in der Dialysebehandlungseinheit angeordnete Steuerungs- und/oder Regelungseinheit handeln. Genausogut kann es sich dabei auch um eine zentrale Steuerungs- und/oder Regelungseinheit des Dialyseversorgungssystems handeln.

Vorteilhafterweise entfällt somit nach einer Behandlungsprozedur die Reinigung der Dialysemaschine, da sämtliche kontaminierten Teile verworfen werden können. Die Rückkontamination der Ringleitung und der zentralen Versorgungsstation ist ebenfalls minimiert, da durch das Verwerfen des/der Einwegartikel eine Fortsetzung der Kontamination über mehrere Behandlungseinheiten, wie dies beispielsweise bei den bekannten Dialysemaschinen aus dem Stand der Technik der Fall ist, nicht möglich ist.

Erfindungsgemäß ist vorgesehen, dass das wenigstens eine Hydraulik-Modul als ausschließliches Mittel zur Führung, Förderung, Verarbeitung, Mischung und/oder Bilanzierung der Dialyseflüssigkeit in der Dialysebehandlungseinheit vorgesehen ist.

Ferner ist möglich, dass als Mittel zur Führung, Förderung und/oder Verarbeitung des Blutes in der Dialysebehandlungseinheit wenigstens ein Blut-Modul vorgesehen ist und dass das Blut-Modul als Einwegartikel bzw. Disposable ausgeführt ist.

Es ergibt sich insbesondere der Vorteil, dass aufgrund der Verwendung von disposablen Hydraulik-Modulen nunmehr Disposables sowohl auf der Blut- als auch auf der Dialysatseite möglich sind. Dies erlaubt einen schnellen und einfachen Patientenwechsel, da die Desinfektion deutlich erleichtert und vereinfacht werden kann, weil ein Großteil der kontaminierten Verbrauchsmaterialien nur einmal verwendet wird. Da die Materialien, die auf der Behandlungsgeräteseite in Kontakt mit potentiell infektiösem Material kommen, nach der Behandlung entsorgt werden, muss nur noch die zentrale Dialysatzuleitung bzw. Ringleitung desinfiziert werden. Hauptquelle eines Keimwachstums in der Dialysatzuleitung ist die flüssigkeitsgefüllte Leitung selbst, nicht mehr der Patient. Damit kann die Frequenz der Desinfektion deutlich unter der Frequenz des Patientenwechsels liegen. Hierdurch können Zeit und Kosten eingespart werden. Automatische Verfahren zur Desinfektion von Ringleitungen können hierzu angewandt werden.

Ein Übertritt von Keimen des Patienten auf die Dialysatzuleitung bzw. in das Ringleitungssystem wird bereits allein durch die Flussrichtung minimiert. Die Filter zwischen Zuleitung und Blutleitung schützen in erster Linie den Patienten vor dem Übertritt von Keimen und Endotoxinen, also von Keimen produzierten Giftstoffen aus der Dialysatzuleitung in das Blut. Das gebrauchte und damit potentiell kontaminierte Dialysat wird über eine zweite, in den vorstehenden Figuren nicht eingezeichnete Leitung entsorgt.

Allen möglichen Ausführungsformen der Erfindung ist gemeinsam, dass zwischen Dialysat und Blut wenigstens eine Trennmembran bzw. ein Filter vorgesehen ist, mittels dessen der Übertritt von Keimen und Endotoxinen in den Patienten verhinderbar ist. Mittels einer zweiten Trennmembran bzw. einem zweiten Filter kann einem möglichen Versagen des ersten Filters bzw. der ersten Trennmembran vorgebeugt werden, wie dies beispielsweise bei einer Ruptur des ersten Filters der Fall sein könnte. Wenn es sich um eine einfache Hämodialysebehandlung handelt, bei der der Stoffaustausch zwischen Blut und Dialysat nur über die Dialysatormembran, also über einen Filter erfolgt, so kann es vorteilhafterweise ausreichen, nur einen Ultrafilter einzusetzen. Bei Hämodiafiltrationsbehandlungen wird ein Teil des Austauschvolumens direkt in das Blut gegeben, wobei diese zugegebene Flüssigkeitsmenge im Austausch über den Dialysator wieder entzogen wird. Folglich ergibt sich hieraus die Notwendigkeit eines zweiten Filters, der das direkt in das Blut gegebene Dialysat vor dessen Zugabe in das Blut filtert.

Eine zweistufige Filtration des Dialysats ist auch für Verfahren des sogenannten "online priming" notwendig. Hierbei muss vor Beginn einer Dialysebehandlung der blutführende Fluidteil des Disposables mit einer Spülflüssigkeit ausgewaschen werden. Als Spülflüssigkeit eignet sich Dialysat. In gängigen "priming" Verfahren wird der blutführende Teil des extrakorporalen Kreislaufs mit einer Dialysatquelle und Pumpenmittel verbunden, so dass dieser durchspült werden kann. Beim "online priming" wird die Spülflüssigkeit, z. B. Dialysat, durch entsprechende Pumpenmittel und Ventilstellungen, über den Blutbehandlungsfilter in den blutführenden Teil des extrakorporalen Kreislaufs gedrückt. Anschließend wird der blutführende Teil des extrakorporalen Kreislaufs gespült. Durch Filtration an dem Blutbehandlungsfilter wird der blutführende Teil des extrakorporalen Kreislaufs bereits vor Kontamination geschützt. Allerdings sind zur Sicherheit immer zwei Filtrationsstufen zu durchlaufen, wenn Flüssigkeit in den blutführenden Teil übertreten soll. Stromauf vom Blutbehandlungsfilter ist daher im Dialysat führenden Teil beim "online priming" eine weitere Filterstufe eingebaut.

Da sämtliche Fluidkreisläufe der Dialysebehandlungseinheit als Einwegartikel ausgeführt sind, kann eine einfache und sichere Durchführung von Dialysebehandlungen gewährleistet werden. Weiter ergibt sich als Vorteil, dass die Abfolge der Dialysebehandlungen erhöht bzw. die Abstände zwischen den Behandlungen verkürzt werden können, da nunmehr auf eine Reinigungs- und/oder Desinfektionsprozedur verzichtet werden kann. Stattdessen erfolgt eine erneute Aufrüstung mit einem Hydraulik-Modul und den weiteren Bestandteilen eines Schlauchsets, die steril verfügbar und bereitstellbar sind.

Des Weiteren kann vorgesehen sein, dass wenigstens ein Filter zur Vermeidung von Rückkontamination und/oder zur Filtration der Dialyseflüssigkeit vorgesehen ist.

Ferner ist möglich, dass der wenigstens eine Filter ein mehrfach verwendbarer Filter ist, der in Dialysatflussrichtung stromaufwärts des Hydraulik-Moduls und Blut-Moduls angeordnet ist.

Vorteilhaft ist es, wenn der wenigstens eine Filter ein Einwegartikel ist.

Möglich ist weiter, dass der wenigstens eine Filter ein Bestandteil des Hydraulik-Moduls und/oder des Blut-Moduls ist.

Darüber hinaus kann vorgesehen sein, dass wenigstens ein erster Filter und wenigstens ein zweiter Filter vorgesehen sind.

Es ist möglich, dass der wenigstens eine erste Filter ein mehrfach verwendbarer Filter ist, der in Dialysatflussrichtung stromaufwärts des Hydraulik-Moduls und Blut-Moduls angeordnet ist.

Vorzugsweise ist der wenigstens eine erste Filter ein Einwegartikel.

Weiter vorteilhaft kann vorgesehen sein, dass der wenigstens eine zweite Filter ein Einwegartikel ist.

Es ist denkbar, dass der wenigstens eine zweite Filter ein Bestandteil des Hydraulik-Moduls und/oder des Blut-Moduls ist.

Des Weiteren kann vorgesehen sein, dass sowohl das Hydraulik-Modul als auch das Blut-Modul jeweils einen Filter aufweist, der Bestandteil des Hydraulik-Moduls bzw. des Blut-Moduls ist.

Darüber hinaus ist möglich, dass der Filter ein Ultrafilter ist, insbesondere ein Ultrafilter in Form eines Hohlfaserfilters ist, wobei die Membran des Filters vorzugsweise eine mittlere Porengröße von ca. 0,1-0,5 µm aufweist, besonders bevorzugt eine mittlere Porengröße von ca. 0,2 µm aufweist.

Außerdem kann vorgesehen sein, dass von dem wenigstens einen Leitungsmittel, mittels dessen Dialyseflüssigkeit leitbar ist, wenigstens drei dialysatführende Leitungen abzweigen und dass das Ventilsystem wenigstens drei Ventile aufweist, wobei jeweils ein Ventil in einer dialysatführenden Leitung angeordnet ist, mittels derer Dialysat zur Dialysebehandlungseinheit zuführbar ist.

Bei den dialysatführenden Leitungen kann es sich um Leitungen des Leitungsmittels handeln, an das die Dialysebehandlungseinheit anschließbar ist und mittels dessen Dialyseflüssigkeit, aber in einem Desinfektionsmodus auch Desinfektionsflüssigkeit leitbar ist.

Denkbar ist ferner, dass zwei dialysatführende Leitungen an einer ersten Seite des Filters an einen ersten Raum des Filters angeschlossen und/oder anschließbar sind, wobei der erste Raum des Filters von einem zweiten Raum des Filters durch die Membran des Filters getrennt ist und wobei die dritte dialysatführende Leitung auf einer zweiten Seite des Filters an den zweiten Raum des Filters angeschlossen und/oder anschließbar ist, so dass mittels der weiteren dritten Leitung die beiden ersten Leitungen umgehbar sind bzw. dass die dritte Leitung zu den beiden ersten Leitungen eine Bypass-Leitung ausbildet.

Weiter ist möglich, dass mittels der wenigstens drei Ventile eine Ventilanordnung geschaffen wird, wobei mittels einer Steuerungs- und/oder Regelungseinheit die Ventilanordnung derart betreibbar und/oder schaltbar ist, dass mittels der Ventilanordnung der Filter auf einer oder beiden Seiten und/oder durch die Membran des Filters hindurch desinfizierbar ist.

Des Weiteren kann vorgesehen sein, dass mittels einer Steuerungs- und/oder Regelungseinheit die Ventilanordnung derart betreibbar und/oder schaltbar ist, dass mittels der Ventilanordnung der Filter abwechselnd von beiden Seiten der Membran des Filters desinfizierbar und mit Desinfektionsflüssigkeit durchströmbar ist.

Darüber hinaus ist denkbar, dass in einem ersten Modus ein erstes Ventil der zwei Ventile, die in den dialysatführenden Leitungen, die auf einer ersten Seite des Filters anschließbar oder angeschlossen sind, angeordnet sind, in einen geöffneten Zustand überführbar ist, während das zweite Ventil der zwei Ventile geschlossen oder schließbar ist, und das dritte Ventil, das in der weiteren dritten dialysatführenden Leitung, die auf der zweiten Seite des Filters anschließbar oder angeschlossen ist, angeordnet ist, schließbar oder geschlossen ist, und dass in einem zweiten Modus das erste Ventil der zwei Ventile schließbar oder geschlossen ist, während das zweite Ventil der zwei Ventile in einen geöffneten Zustand überführbar ist, und dass das dritte Ventil in einen geöffneten Zustand überführbar ist.

Des Weiteren kann vorgesehen sein, dass die Dialysebehandlungseinheit durch wenigstens ein viertes Ventil von der wenigstens einen dialysatführenden Leitung, die Desinfektionsflüssigkeit führt oder mittels derer Desinfektionsflüssigkeit führbar ist, trennbar ist.

Ferner ist möglich, dass das vierte Ventil stromaufwärts des Hydraulik-Moduls und stromabwärts des Filters in einer an der zweiten Seite des Filters angeschlossenen und/oder anschließbaren dialysatführenden Leitung angeordnet ist und wobei der Filter zwischen wenigstens zwei Ventilen des Ventilsystems angeordnet ist, wobei vorzugsweise zwei Ventile auf der ersten Seite des Filters und zwei Ventile auf der zweiten Seite des Filters angeordnet sind.

Die erste Seite des Filters kann die Seite sein, die dem Hydraulik-Modul abgewandt ist, d.h. dass das Dialysat noch über die Membran des Filters übertreten muss, um zum Hydraulik-Modul zu gelangen. Die zweite Seite des Filters ist dementsprechend die dem Hydraulik-Modul zugewandte Seite. Dadurch ergibt sich der Vorteil, dass mit der dritten Leitung eine Leitung zur Verfügung steht, die in einem Desinfektionsmodus für das Leitungsmittel verwendbar ist. So kann beispielsweise der erste Modus der normale Behandlungsmodus sein, wobei die erste Leitung zur Zuführung des Dialysates dient und die zweite und dritte Leitung über die dortigen Ventile gesperrt sind. Im zweiten Modus, der ein Reinigungs- und/oder Desinfektionsmodus ist, wird die erste Leitung über das dort befindliche Ventil gesperrt, wobei in vorteilhafter Ausgestaltung auch die Anschlussleitung zur Dialysebehandlungseinheit, mit der die Dialysebehandlungseinheit an das Leitungsmittel angeschlossen ist, über ein dort befindliches viertes Ventil gesperrt wird. Die zweite und dritte Leitung werden in diesem Modus geöffnet, so dass der Filter zu beiden Seiten der Membran angeströmt werden kann. Eine besonders vorteilhafte Reinigung und/oder Desinfektion ist hierdurch möglich und der Filter kann unproblematisch als mehrfach verwendbarer Filter ausgeführt sein. Die Ansteuerung der Ventile kann beispielsweise über eine Steuerungs- und/oder Regelungseinheit des Leitungsmittels erfolgen. Diese Steuerungs- und/oder Regelungseinheit kann die zentrale Steuerungs- und/oder Regelungseinheit des Leitungsmittels sein.

Ein disposables Schlauchset kann wenigstens ein Hydraulik-Modul gemäß der Erfindung umfassen, wobei vorzugsweise das Blut-Modul ebenfalls in das Schlauchset integriert ist.

Das Schlauchset ist vorteilhafterweise für den Einsatz in einer Dialysebehandlungseinheit vorgesehen, wobei es sich in vorteilhafter Ausgestaltung um eine Dialysebehandlungseinheit eines Dialyseversorgungssystems nach Anspruch 1 bis 14 handelt. Vorzugsweise ist die Dialysebehandlungseinheit mit entsprechenden Aufnahmen versehen, an die das Schlauchset mittels entsprechender Gegenstücke anschließbar ist.

Eine disposable Kassette kann wenigstens ein Hydraulik-Modul gemäß der Erfindung umfassen, wobei vorzugsweise das Blut-Modul ebenfalls in die Kassette integriert ist.

Die Kassette ist vorteilhafterweise für den Einsatz in einer Dialysebehandlungseinheit vorgesehen, wobei es sich in vorteilhafter Ausgestaltung um eine Dialysebehandlungseinheit eines Dialyseversorgungssystems nach Anspruch 1 bis 14 handelt. Vorzugsweise ist die Dialysebehandlungseinheit mit entsprechenden Aufnahmen versehen, an die bzw. in die die Kassette mittels entsprechender Gegenstücke anschließbar ist.

Das Hydraulik-Modul, insbesondere das Hydraulik-Modul des Schlauchsets und/oder das Hydraulik-Modul der Kassette, weist insbesondere sämtliche Funktionen bzw. funktionalen Elemente für die Verarbeitung der Dialyseflüssigkeit auf, im Einzelnen eine Bilanziereinheit zur Bilanzierung von frischem und verbrauchtem Dialysat, optional eine Heizung zur Erwärmung des frischen Dialysats, einen Drucksensor, einen Temperatursensor, einen Blutleckdetektor, optional eine Sensoreinheit zur Bestimmung der Clearance und optional ein Pumpenmittel zur Förderung des Dialysats und des Ultrafiltrats. Die Sensoreinheit bzw. sonstige eingesetzte Sensoren müssen nicht notwendigerweise Bestandteil des Hydraulik-Moduls sein, sondern können in vorteilhafter Ausgestaltung auch Bestandteil der Dialysebehandlungseinheit sein. Das als Disposable ausgeführte Hydraulik-Modul weist sodann die entsprechenden Schnittstellen zu den Sensoren bzw. der Sensoreinheit auf.

Das Blut-Modul, insbesondere das Blut-Modul des Schlauchsets und/oder das Blut-Modul der Kassette, weist in vorteilhafter Ausgestaltung sämtliche Funktionen bzw. funktionalen Elemente für die Verarbeitung des zu behandelnden Patientenblutes auf, im Einzelnen einen Bluttemperaturmonitor, einen Temperatursensor, eine Sensorik zur Analyse der Zusammensetzung des Blutes, z. B. hinsichtlich Hämoglobin, Plasmaanteil, Blutvolumen, eine Zugabemöglichkeit für Antikoagulanz, einen Gasblasendetektor, ein Flüssigkeitsdetektor und eine Ultraschallsensorik. Die Sensoreinheit bzw. sonstige eingesetzte Sensoren müssen nicht notwendigerweise Bestandteil des Blut-Moduls sein, sondern können in vorteilhafter Ausgestaltung auch Bestandteil der Dialysebehandlungseinheit sein. Das vorteilhafterweise als Disposable ausgeführte Blut-Modul weist sodann die entsprechenden Schnittstellen zu den Sensoren bzw. der Sensoreinheit auf.

In das Blut-Modul kann in vorteilhafter Ausgestaltung der Dialysebehandlungsfilter integriert sein.

Die Dialysebehandlungseinheit ist insbesondere eine Dialysemaschine oder ein Dialysebehandlungsmonitor.

Weitere Einzelheiten und Vorteile der Erfindung sollen nun anhand eines in der Zeichnung dargestellten Ausführungsbeispiels näher erläutert werden.

Es zeigen:
- Figur 1:: eine schematische Darstellung eines herkömmlichen Dialyseversorgungssystems;
- Figur 2:: eine schematische Darstellung eines herkömmlichen CRRT-Systems;
- Figur 3:: eine schematische Darstellung eines "bedside monitor" Systems;
- Figur 4:: eine schematische Darstellung einer Ausführungsform eines erfindungsgemäßen Dialyseversorgungssystems;
- Figur 5:: eine schematische Darstellung einer möglichen Ausgestaltung der in Figur 4 gezeigten Ausführungsform;
- Figur 6:: eine schematische Darstellung einer weiteren möglichen Ausgestaltung der in Figur 4 gezeigten Ausführungsform;
- Figur 7:: eine schematische Darstellung einer weiteren möglichen Ausgestaltung der in Figur 4 gezeigten Ausführungsform; und
- Figur 8:: eine schematische Darstellung einer weiteren möglichen Ausgestaltung der in Figur 4 gezeigten Ausführungsform.

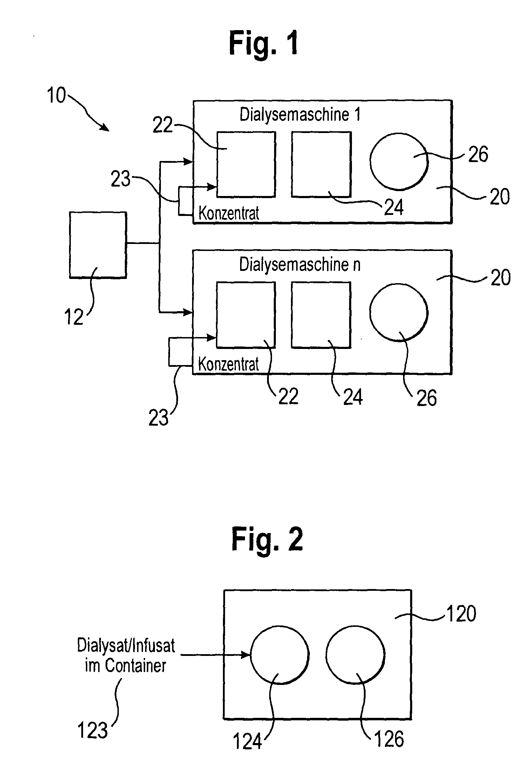

Die Figuren 1, 2 und 3 zeigen in schematischer Darstellung ein herkömmliches Dialyseversorgungssystem, ein herkömmliches CRRT-System und ein bekanntes "bedside monitor" System. Dabei sind disposable Module der in Figur 1 bis 3 gezeigten Systeme 10, 120 und 200 mittels eines kreisförmigen Symbols dargestellt.

Bei einem herkömmlichen Dialyseversorgungssystem 10 gemäß Figur 1 ist üblicherweise ein zentraler Versorgungsteil 12 und wenigstens eine, zumeist mehrere Dialysemaschinen 20, also n Dialysemaschinen 20 vorgesehen. Die Zufuhr von frischem Wasser erfolgt üblicherweise zentral durch eine stationäre Umkehrosmoseanlage bzw. Reinstwasseranlage. Demgegenüber erfolgt die Dialysatzubereitung, die Kontrolle der Ultrafiltration und die Etablierung des extrakorporalen Blutkreislaufs dezentral mittels einer separaten Behandlungseinheit 20, insbesondere mittels einer Dialysemaschine 20.

Hierzu ist beispielsweise in der Dialysemaschine ein Dialysatmodul 22 insbesondere für die Dialysatzubereitung, ein Hydraulik-Modul 24 insbesondere für die Kontrolle der Ultrafiltration und ein Blut-Modul 26 insbesondere zur Etablierung des extrakorporalen Blutkreislaufs vorgesehen. So kann beispielsweise mittels des Dialysatmoduls 22 durch die Vermischung von Konzentrat 23 und dem zugeführten Frischwasser das in der Dialysemaschine benötigte Dialysat zubereitet werden.

Dabei sind sämtliche Fluidwege bzw. Fluidführungen auf der Dialysatseite des Dialyseversorgungssystem 10, also insbesondere auch die Fluidwege des Dialysatmoduls 22 und des Hydraulik-Moduls 24 zur mehrfachen Verwendung vorgesehen. Dementsprechend ist in regelmäßigen Abständen eine Reinigung, Desinfektion und auch eine Entkalkung erforderlich.

Der extrakorporale Blutkreislauf im Blut-Modul 26 ist jedoch zur einmaligen Verwendung vorgesehen und daher regelmäßig als Disposable ausgeführt.

Wie in Figur 2 gezeigt, kann bei Behandlungseinheiten für die kontinuierliche Nierenersatztherapie (continuous renal replacement therapy - CRRT), kurz CRRT-Behandlungseinheiten genannt, der zentrale Versorgungsteil 12 gemäß Figur 1 fehlen. Stattdessen werden Dialysatcontainer 123 oder Infusatcontainer 123 eingesetzt. Denn diese Behandlungseinheiten 120 kommen in einem Behandlungsumfeld zum Einsatz, bei dem Ort und Häufigkeit der Anwendung stark variieren. Haupteinsatzgebiet ist beispielsweise die Behandlung von akutem Nierenversagen von Intensivpatienten. Der Einsatz auf einer Intensivstation stellt dabei weiter hohe Anforderungen an die Hygiene und damit an die Desinfizierbarkeit dieser Behandlungseinheiten. Daher haben sich für diese Behandlungseinheiten disposable Hydraulik-Module 124 und disposable Blut-Module 126 etabliert. So kann eine schnelle und zuverlässige Aufrüstung der Behandlungseinheit 120 auch nach einer längeren Standzeit erfolgen, da keinerlei mehrfach verwendbare Fluidwege bei der Behandlungseinheit 120 vorhanden sind und diese dementsprechend auch nicht gereinigt, desinfiziert oder entkalkt werden müssen.

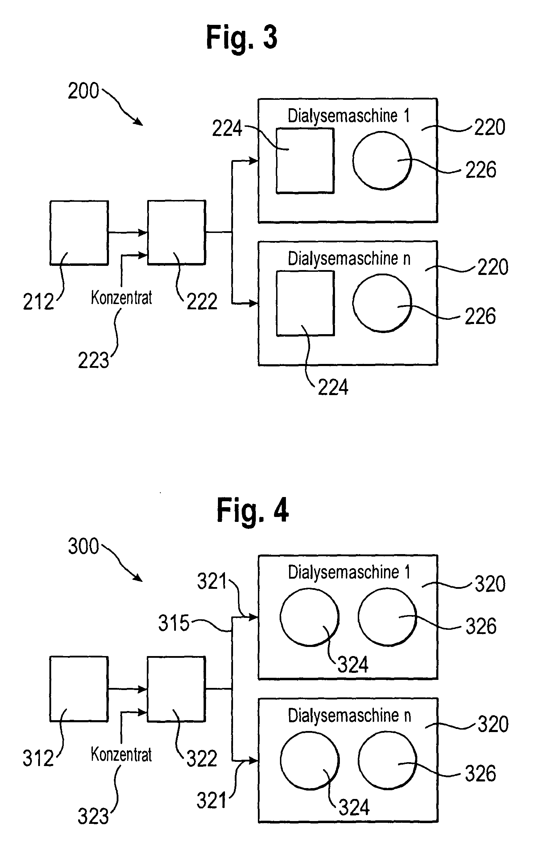

Figur 3 zeigt ein bekanntes "bedside" System 200 mit einem zentralen Versorgungsteil 212 und einer zentralen Dialysatzubereitung 222, in der Konzentrat 223 und Frischwasser zu Dialysat vermischt werden. Die Dialysemaschinen 220, die in einer Anzahl n vorgesehen sein können, weisen jeweils ein Hydraulik-Modul 224 und ein Blut-Modul 226 auf, die vergleichbar oder baugleich den in Figur 1 gezeigten und in diesem Zusammenhang beschriebenen Hydraulik-Modulen 24 und Blut-Modulen 26 ausgeführt sein können. Auch hier werden sämtliche Fluidführungswege auf der Dialysatseite des "bedside" Systems 200 mehrfach verwendbar ausgeführt. Daher ist auch hier insbesondere das Hydraulik-Modul 226 in regelmäßigen Abständen zu reinigen, zu desinfizieren und zu entkalken.

Figur 4 zeigt als ein erstes Ausführungsbeispiel der Erfindung ein zentrales Dialyseversorgungssystem 300. Dabei ist ein zentraler Versorgungsteil 312 und eine zentrale Dialysatzubereitung 322 vorgesehen, in der Konzentrat 323 und Frischwasser zu Dialysat vermischt werden.

Die disposablen Module der in Figur 4 bis 8 gezeigten Systeme 300 sind grundsätzlich mittels eines kreisförmigen Symbols dargestellt.

Dialysat kann vorzugsweise als Fluid definiert werden, das wenigstens 50 % des Anteils an Natrium und Bikarbonat aufweist, der für eine Dialysebehandlung benötigt wird.

Die als Dialysemaschinen 320 ausgeführten Behandlungseinheiten werden über entsprechende Anschlüsse, die durch die Pfeile 321 angedeutet werden, an die Verbindungsleitung angeschlossen, mittels derer die Dialysemaschinen 320 mit Dialysat versorgt werden können. Die Verbindungsleitung 315 kann beispielsweise eine stationäre Ringleitung 315 sein.

Vorteilhafterweise sind nun sämtliche Fluidführungsmittel 324 und 326, also das Hydraulik-Modul 324 und das Blut-Modul 326, die zumindest mittelbar in Kontakt mit dem Patienten bzw. dessen Blut sind, als Einwegartikel, also disposabel ausgeführt.

Das Hydraulik-Modul 324 weist in vorteilhafter Ausgestaltung sämtliche Funktionen bzw. funktionalen Elemente für die Verarbeitung der Dialyseflüssigkeit auf, im Einzelnen eine Bilanziereinheit zur Bilanzierung von frischem und verbrauchtem Dialysat, optional eine Heizung zur Erwärmung des frischen Dialysats, einen Drucksensor, einen Temperatursensor, einen Blutleckdetektor, optional eine Sensoreinheit zur Bestimmung der Clearance und optional ein Pumpenmittel zur Förderung des Dialysats und des Ultrafiltrats.

Das Blut-Modul 326 weist in vorteilhafter Ausgestaltung sämtliche Funktionen bzw. funktionalen Elemente für die Verarbeitung zu behandelnden Patientenblutes auf, im Einzelnen einen Bluttemperaturmonitor, einen Temperatursensor, eine Sensorik zur Analyse der Zusammensetzung des Blutes, z. B. hinsichtlich Hämoglobin, Plasmaanteil, Blutvolumen, eine Zugabemöglichkeit für Antikoagulanz, einen Gasblasendetektor, ein Flüssigkeitsdetektor und eine Ultraschallsensorik.

Hinsichtlich der Sensorik und Aktorik kann bei den disposablen Hydraulik-Modulen 324 und Blut-Modulen 326 vorgesehen sein, dass hier nur die Anschlüsse an die Sensorik und Aktorik disposabel ausgeführt ist.

Die Module 324 und 326 können aufgrund der Verarbeitung unterschiedlicher Fluide als getrennte Module 324 und 326 angesehen werden. Gegenständlich können diese jedoch auf einer oder mehrerer integral ausgeführten Einheiten vorliegen. So können die Module 324 und 326 zu einer disposablen Kassette zusammengefasst sein. Denkbar ist auch, die Module 324 und 326 zu einem integralen und disposablen Schlauchset umfassend beide Module 324 und 326 zusammenzufassen.

Der nicht näher dargestellte Dialysator und die Infusionsstelle des Substituats für Hämofiltrations- oder Hämodiafiltrationstherapien sind dann beispielsweise modulübergreifende Schnittstellen, die von beiden Modulen 324 und 326 angeströmt werden müssen.

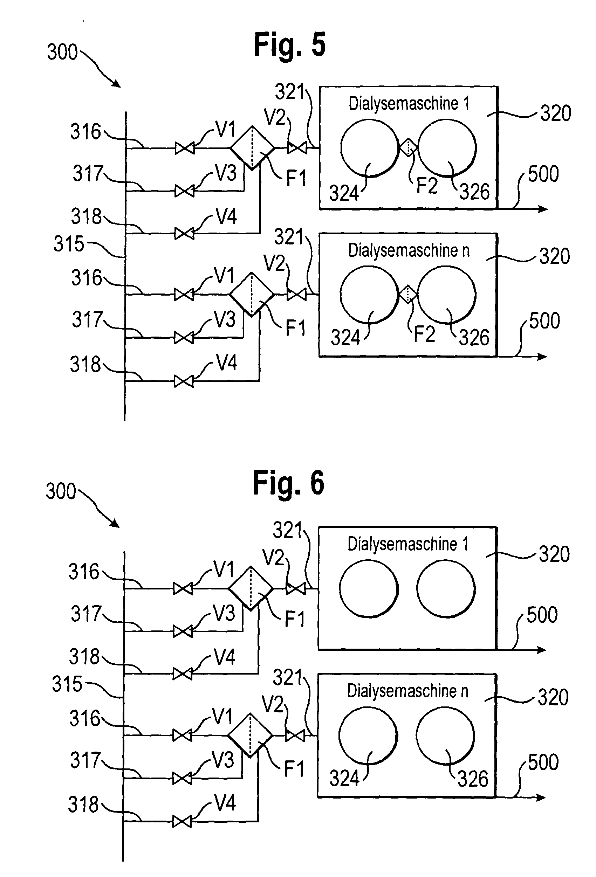

Figur 5 zeigt eine erst optionale Ausgestaltung der in Figur 4 gezeigten Ausführungsform des Dialyseversorgungssystem 300 mit den Dialysemaschinen 320.

Im Ringleitungssystem 315 ist ein Ventilsystem umfassend die Ventile V1, V3 und V4 vorgesehen, mit denen der Zulauf des Dialysates einstellbar ist. In der Leitung 315 wird als integraler Bestandteil der Ringleitung 315 ein wiederverwendbarer bzw. mehrfach verwendbarer Filter F1 vorgesehen, der vorzugsweise erst nach einer gewissen Standzeit zu wechseln bzw. zu reinigen ist. Der Filter F1 trennt dabei die Leitungen 316 und 317 mit den Ventilen V1 und V3 von der Leitung 321 mit dem Ventil V2. Mittels der Leitung 318 kann ein Bypass zu den Leitungen 316 und 317 geschaffen werden, wobei die Leitung 318 über das Ventil V4 gesperrt werden kann und üblicherweise, vor allem aber im Behandlungsbetrieb auch gesperrt ist.

Die Leitungen 316, 317, 318 mit den Ventilen V1, V2, V3, V4 sowie dem Filter F1 sind analog und vorzugsweise baugleich für alle Dialysemaschinen 320 vorgesehen.

Des Weiteren ist zwischen dem Hydraulik-Modul 324 und dem Blut-Modul 326 ein weiterer, disposabler und einmal verwendbarer Filter F2 vorgesehen.

Das Dialyseversorgungssystem 300 erlaubt es beispielsweise, unproblematisch einmal täglich, vorzugsweise nachts eine Desinfektion des Ringleitungssystem 315 vorzunehmen.

Eine erste Sicherheitsstufe bildet dabei bereits der disposable Filter F2. Eine weitere Sicherheitsstufe wird ferner durch den Filter F1 ausgebildet, der nach jedem Desinfektionsvorgang hinsichtlich seiner Funktion überprüft wird.

Für einen Behandlungsvorgang mit konnektierter Dialysemaschine 320 über die Leitung 321 an das Ringleitungssystem 315 werden die Ventile V1 und V2 geöffnet, um der Dialysemaschine 320 Dialysat zuführen zu können. Zumindest ein Teil dieses Fluidstroms oder auch der gesamte Fluidstrom kann durch den Filter F2 strömen und kann für den Modus Priming ("priming mode"), Spülen ("rinse back"), Hämodiafiltration ("HDF") oder Hämofiltration ("HF") verwendet werden. Der ggf. übrige Teil oder der gesamte Fluidstrom, der durch den Filter F2 geströmt ist, kann als Dialysat verwendet werden.

Die Ventile V3 und V4 können im Desinfektionsmodus zur Desinfektion des Ringleitungssystems 315 benutzt werden. Sie werden vorzugsweise über eine zentrale Steuerungs- und Regelungseinheit der Ringleitung 315 angesteuert. Das Ventil V2 ist bei der Desinfektion des Ringleitungssystems 315 in der Regel geschlossen.

Figur 6 zeigt eine Abwandlung der in Figur 5 gezeigten Ausführungsform. Identische Merkmale sind dabei mit identischen Bezugszeichen versehen. Die in Figur 6 gezeigte Ausführungsform erlaubt nur eine Hämodialysebehandlung.

Hier fehlt der disposable Filter F2, der gemäß dem in Figur 5 gezeigten Ausführungsbeispiels zwischen dem Hydraulik-Modul 324 und dem Blut-Modul 326 angeordnet ist. Ein Priming kann hier ebenfalls online erfolgen, nämlich indem der Filter F1 als erster Ultrafilter und sodann auch der Dialysator als weiterer Ultrafilter eingesetzt wird. Das Dialysat wird von der Dialysatseite über die semipermeable Membran des Dialysators auf die Blutseite gedrückt, so dass ein Priming erfolgen kann.

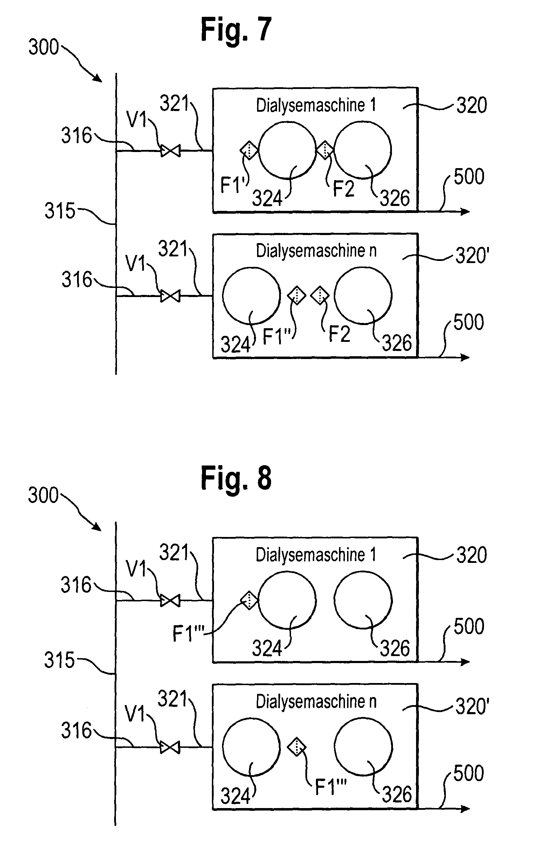

Figur 7 zeigt eine Abwandlung der in Figur 5 und 6 gezeigten Ausführungsform. Identische Merkmale sind dabei mit identischen Bezugszeichen versehen.

Hier wird der wiederverwendbare Filter F1 aus dem in Figur 5 und 6 gezeigten Ausführungsbeispiel durch einen weiteren disposablen Einmalfilter F1' ersetzt. Somit sind hier wenigstens zwei Einmalfilter F1' und F2 vorgesehen, die jeweils als Ultrafilter ausgeführt sind. Die Position dieser Einmalfilter F1' und F2 kann stromaufwärts und/oder stromabwärts des Hydraulik-Moduls 324 sein.

Die obere Dialysemaschine 320 zeigt eine erste Anordnung, bei der der Einmalfilter F1' stromaufwärts des Hydraulik-Moduls 324 und der zweite Einmalfilter F2 stromabwärts des Hydraulik-Moduls 324 angeordnet ist.

Die untere Dialysemaschine 320' zeigt eine zweite Anordnung, bei der beide Filter F1" und F2 stromabwärts des Hydraulik-Moduls 324 angeordnet sind. Der Einmalfilter F1" ist also stromabwärts des Hydraulik-Moduls 324 und der zweite Einmalfilter F2 stromaufwärts des Blut-Moduls 326 angeordnet.

Figur 8 zeigt eine Abwandlung der in Figur 7 gezeigten Ausführungsform. Identische Merkmale sind dabei mit identischen Bezugszeichen versehen.

Hier wird analog zu dem in Figur 6 dargestellten Ausführungsbeispiel einer der beiden Ultrafilter weggelassen, so dass nur ein Betrieb im Modus Hämodialysebehandlung erfolgen kann.

Ein Priming kann hier ebenfalls online erfolgen, nämlich indem der Filter F1'" als erster Ultrafilter und sodann auch der Dialysator als weiterer Ultrafilter eingesetzt wird. Das Dialysat wird von der Dialysatseite über die semipermeable Membran des Dialysators auf die Blutseite gedrückt, so dass ein Priming erfolgen kann.

Die obere Dialysemaschine 320 zeigt eine erste Anordnung, bei der der Einmalfilter F1'" stromaufwärts des Hydraulik-Moduls 324 angeordnet ist.

Die untere Dialysemaschine 320' zeigt eine zweite Anordnung, bei der der Einmalfilter F1'" stromabwärts des Hydraulik-Moduls 324 angeordnet ist.

Die Figuren 5 bis 8 zeigen somit unterschiedliche Anordnungen von Filtern F1, F1', F1", F1"', F2, die bezogen auf die Strömungsrichtung der frischen Dialyseflüssigkeit gesehen, zwischen und/oder vor den einzelnen Modulen 324 und 326 angeordnet werden. Es handelt sich bei den Filtern F1, F1', F1", F1"', F2 um Ultrafiltrationsfilter, die in der Lage sind, angeliefertes Dialysat zu dekontaminieren. Etabliert sind für solche Prozesse Filter unterschiedlicher Art, vorzugsweise Hohlfasermodule. Die mittlere Porengröße der Filter kann ca. 0,2 µm betragen.

Für Hämodiafiltrationsbehandlungen wird vorhandenes Substituat, das die gleiche Zusammensetzung wie in der Hämodialyse verwendetes Dialysat aufweist, in die Blutleitungsbahnen des extrakorporalen Kreislaufs infundiert. Bevorzugt werden die Tropfkammern des Blutkreislaufs für diese Infusion verwendet. Die Zugabe von Substituat in das Blutschlauchsystem kann vor und/oder nach dem Filter erfolgen. Es besteht die Möglichkeit, das Substituat direkt, also online, aus dem Dialysat durch einen zusätzlichen Filtrationsschritt herzustellen. Dies wird bei der online Hämodiafiltration (online HDF) angewandt, wie dies beispielsweise mit den in Figur 5 und 7 gezeigten Systemen möglich ist.

Grundsätzlich können zusätzlich, was nicht näher in den Figuren 5 bis 8 dargestellt ist, die Dialysebehandlungsfilter bzw. Dialysatoren im Blut-Modul 326 integriert sein.

Die in den Figuren 5 bis 8 dargestellten Dialysemaschinen 320 bzw. 320' weisen zur Abführung des verbrauchten Dialysats Abführleitungen 500 auf, die entweder zu einer zentralen, nicht näher dargestellten Abführleitung oder zu einem Sammelbehältnis wie z. B. einem Beutel für die verbrauchte Dialyselösung führen.

## Patentansprüche

1. Dialyseversorgungssystem (300)
- mit einer zentralen Herstellungseinheit, mittels derer Dialyseflüssigkeit herstellbar ist,
- mit einem Leitungsmittel (315), mittels dessen Dialyseflüssigkeit leitbar ist,
- mit mehreren Dialysebehandlungseinheiten (320, 320')
- mit Anschlussmitteln, mittels denen die Dialysebehandlungseinheiten (320, 320') an das Leitungsmittel (315) anschließbar sind, und
- mit Ventilen (V1, V2, V3, V4), mittels denen der Zufluss von Dialyseflüssigkeit zu den Dialysebehandlungseinheiten (320, 320') regelbar ist,
wobei als einzige Mittel zur Führung der Dialyseflüssigkeit in den Dialysebehandlungseinheiten (320, 320') Hydraulik-Module (324) vorgesehen sind,
**dadurch gekennzeichnet,**
**dass** die Hydraulik-Module (324) als Einwegartikel ausgeführt sind.

2. Dialyseversorgungssystem (300) nach Anspruch 1, **dadurch gekennzeichnet, dass** Filter (F1, F1', F1", F1'"') zur Vermeidung von Rückkontamination und/oder zur Filtration der Dialyseflüssigkeit vorgesehen sind.

3. Dialyseversorgungssystem (300) nach Anspruch 2, **dadurch gekennzeichnet, dass** die Filter mehrfach verwendbare Filter (F1) sind, die in Dialysatflussrichtung stromaufwärts der Hydraulik-Module (324) angeordnet sind.

4. Dialyseversorgungssystem (300) nach Anspruch 2, **dadurch gekennzeichnet, dass** die Filter (F1', F1", F1"', F2) Einwegartikel sind.

5. Dialyseversorgungssystem nach einem der Ansprüche 2 oder 4, **dadurch gekennzeichnet, dass** die Filter (F1', F1", F1'", F2) Bestandteile der Hydraulik-Module (324) sind.

6. Dialyseversorgungssystem (300) nach Anspruch 2, **dadurch gekennzeichnet, dass** für die Dialysebehandlungseinheiten (320, 320') jeweils wenigstens ein erster Filter (F1, F1', F1") und wenigstens ein zweiter Filter (F2) vorgesehen sind.

7. Dialyseversorgungssystem (300) nach Anspruch 6, **dadurch gekennzeichnet, dass** der wenigstens eine erste Filter (F1) ein mehrfach verwendbarer Filter (F1) ist, der in Dialysatflussrichtung stromaufwärts des jeweiligen Hydraulik-Moduls (324) angeordnet ist.

8. Dialyseversorgungssystem (300) nach Anspruch 6, **dadurch gekennzeichnet, dass** der wenigstens eine erste Filter (F1', F1") ein Einwegartikel ist.

9. Dialyseversorgungssystem (300) nach einem der Ansprüche 6 bis 8, **dadurch gekennzeichnet, dass** der wenigstens eine zweite Filter (F2) ein Einwegartikel ist.

10. Dialyseversorgungssystem (300) nach einem der Ansprüche 6 bis 9, **dadurch gekennzeichnet, dass** der wenigstens eine zweite Filter (F2) ein Bestandteil des jeweiligen Hydraulik-Moduls (324) ist.

11. Dialyseversorgungssystem nach einem der Ansprüche 2 bis 10, **dadurch gekennzeichnet, dass** die Filter (F1, F1', F1", F1"', F2) Ultrafilter sind.

12. Dialyseversorgungssystem nach Anspruch 11, **dadurch gekennzeichnet, dass** die Filter (F1, F1', F1", F1"', F2) Ultrafilter in Form von Hohlfaserfiltern sind.

13. Dialyseversorgungssystem nach Anspruch 11 oder 12, **dadurch gekennzeichnet, dass** die Membranen der Filter (F1, F1', F1", F1 ''', F2) eine mittlere Porengröße von 0,1 µm bis 0,5 µm aufweisen.

14. Dialyseversorgungssystem nach Anspruch 13, **dadurch gekennzeichnet, dass** die Membranen der Filter (F1, F1', F1", F1"', F2) eine mittlere Porengröße von ca. 0,2 µm aufweisen.

## Claims

1. A dialysis supply system (300)
- having a central manufacturing unit by means of which dialysis liquid can be manufactured,
- having a conducting means (315) by means of which dialysis liquid can be conducted,
- having a plurality of dialysis treatment units (320, 320')
- having connection means by means of which the dialysis treatment units (320, 320') can be connected to the conducting means (315), and
- having valves (V1, V2, V3, V4) by means of which the inflow of dialysis liquid to the dialysis treatment units (320, 320') can be regulated,
wherein hydraulic modules (324) are provided as the only means for the conducting of the dialysis liquid in the dialysis treatment units (320, 320'),
**characterized in that**
the hydraulic modules (324) are configured as single-use articles.

2. A dialysis supply system (300) in accordance with claim 1, **characterized in that** filters (F1, F1', F1", F1"') are provided for avoiding back contamination and/or for filtering the dialysis liquid.

3. A dialysis supply system (300) in accordance with claim 2, **characterized in that** the filters are filters (F1) which can be used a multiple of times and which are arranged upstream of the hydraulic modules (324) in the dialysate flow direction.

4. A dialysis supply system (300) in accordance with claim 2, **characterized in that** the filters (F1', F1 ", F1"', F2) are single-use articles.

5. A dialysis supply system in accordance with one of the claims 2 or 4, **characterized in that** the filters (F1', " F1 ", F1"', F2) are components of the hydraulic modules (324).

6. A dialysis supply system (300) in accordance with claim 2, **characterized in that** at least one first filter (F1, F1', F1") and at least one second filter (F2) are respectively provided for the dialysis treatment units (320, 320').

7. A dialysis supply system (300) in accordance with claim 6, **characterized in that** the at least one first filter (F1) is a filter (F1) which can be used a multiple of times and which is arranged upstream of the respective hydraulic module (324) in the dialysate flow direction.

8. A dialysis supply system (300) in accordance with claim 6, **characterized in that** the at least one first filter (F1', F1") is a single-use article.

9. A dialysis supply system (300) in accordance with one of the claims 6 to 8, **characterized in that** the at least one second filter (F2) is a single-use article.

10. A dialysis supply system (300) in accordance with one of the claims 6 to 9, **characterized in that** the at least one second filter (F2) is a component of the respective hydraulic module (324).

11. A dialysis supply system in accordance with one of the claims 2 to 10, **characterized in that** the filters (F1, F1', F1 ", F1"', F2) are ultrafilters.

12. A dialysis supply system in accordance with claim 11, **characterized in that** the filters (F1, F1', F1 ", F1"', F2) are ultrafilters in the form of hollow fiber filters.

13. A dialysis supply system in accordance with claim 11 or claim 12, **characterized in that** the membranes of the filters (F1, F1', F1 ", F1"', F2) have a mean pore size of 0.1 µm to 0.5 µm.

14. A dialysis supply system in accordance with claim 13, **characterized in that** the membranes of the filters (F1, F1', " F1 ", F1"', F2) have a mean pore size of approximately 0.2 µm.

## Revendications

1. Système d'alimentation pour dialyse (300), comprenant
- une unité de préparation centrale, au moyen de laquelle du liquide de dialyse peut être préparé,
- un moyen formant conduite (315), au moyen duquel du liquide de dialyse peut être transporté,
- plusieurs unités de traitement par dialyse (320, 320')
- des moyens de raccordement, au moyen desquels les unités de traitement par dialyse (320, 320') peuvent être raccordées au moyen formant conduite (315), et
- des vannes (V1, V2, V3, V4), au moyen desquelles l'arrivée de liquide de dialyse vers les unités de traitement par dialyse (320, 320') peut être régulée,
dans lequel des modules hydrauliques (324) sont prévus dans les unités de traitement par dialyse (320, 320') comme unique moyen de guidage du liquide de dialyse,
**caractérisé en ce que**
les modules hydrauliques (324) sont réalisés sous la forme d'articles à usage unique.

2. Système d'alimentation pour dialyse (300) selon la revendication 1, **caractérisé en ce que** des filtres (F1, F1', F1 ", F1"') sont prévus pour éviter une recontamination et/ou pour la filtration du liquide de dialyse.

3. Système d'alimentation pour dialyse (300) selon la revendication 2, **caractérisé en ce que** les filtres sont des filtres (F1) à usage multiple, qui sont disposés en amont des modules hydrauliques (324) dans le sens d'écoulement du dialysat.

4. Système d'alimentation pour dialyse (300) selon la revendication 2, **caractérisé en ce que** les filtres (F1', F1 ", F1"', F2) sont des articles à usage unique.

5. Système d'alimentation pour dialyse selon l'une des revendications 2 ou 4, **caractérisé en ce que** les filtres (F1', F1 ", F1"', F2) font partie des modules hydrauliques (324).

6. Système d'alimentation pour dialyse (300) selon la revendication 2, **caractérisé en ce qu'**au moins un premier filtre (F1, F1', F1") et au moins un second filtre (F2) sont prévus respectivement pour les unités de traitement par dialyse (320, 320').

7. Système d'alimentation pour dialyse (300) selon la revendication 6, **caractérisé en ce que** l'au moins un premier filtre (F1) est un filtre (F1) à usage multiple, qui est disposé en amont du module hydraulique (324) respectif dans le sens d'écoulement du dialysat.

8. Système d'alimentation pour dialyse (300) selon la revendication 6, **caractérisé en ce que** l'au moins un premier filtre (F1', F1") est un article à usage unique.

9. Système d'alimentation pour dialyse (300) selon l'une des revendications 6 à 8, **caractérisé en ce que** l'au moins un second filtre (F2) est un article à usage unique.

10. Système d'alimentation pour dialyse (300) selon l'une des revendications 6 à 9, **caractérisé en ce que** l'au moins un second filtre (F2) fait partie du module hydraulique (324) respectif.

11. Système d'alimentation pour dialyse selon l'une des revendications 2 à 10, **caractérisé en ce que** les filtres (F1, F1', F1 ", F1"', F2) sont des ultrafiltres.

12. Système d'alimentation pour dialyse selon la revendication 11, **caractérisé en ce que** les filtres (F1, F1', F1", F1"', F2) sont des ultrafiltres sous la forme de filtres à fibres creuses.

13. Système d'alimentation pour dialyse selon la revendication 11 ou 12, **caractérisé en ce que** les membranes des filtres (F1, F1', F1 ", F1"', F2) présentent une grosseur de pore moyenne de 0,1 µm à 0,5 µm.

14. Système d'alimentation pour dialyse selon la revendication 13, **caractérisé en ce que** les membranes des filtres (F1, F1', F1 ", F1"', F2) présentent une grosseur de pore moyenne d'environ 0,2 µm.
